(19) Europäisches Patentamt
European Patent Office
Office européen des brevets

(11) **EP 4 052 708 A1**

(12) **EUROPEAN PATENT APPLICATION**
published in accordance with Art. 153(4) EPC

(43) Date of publication:
**07.09.2022 Bulletin 2022/36**

(21) Application number: **20883140.4**

(22) Date of filing: **30.10.2020**

(51) International Patent Classification (IPC):
*A61K 31/60* (2006.01)    *A23K 10/37* (2016.01)
*A23K 20/158* (2016.01)    *A23K 50/10* (2016.01)
*A61K 31/05* (2006.01)    *A61K 36/22* (2006.01)
*A61P 3/10* (2006.01)

(52) Cooperative Patent Classification (CPC):
**A23K 10/37; A23K 20/158; A23K 50/10;
A61K 31/05; A61K 31/60; A61K 36/22; A61P 3/10;
Y02P 60/87**

(86) International application number:
**PCT/JP2020/040763**

(87) International publication number:
**WO 2021/085584 (06.05.2021 Gazette 2021/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.10.2019 JP 2019199095**

(71) Applicant: **Idemitsu Kosan Co., Ltd.
Chiyoda-ku
Tokyo 100-8321 (JP)**

(72) Inventor: **HIKITA, Chie
Tsukuba-shi, Ibaraki 300-2646 (JP)**

(74) Representative: **Vossius & Partner
Patentanwälte Rechtsanwälte mbB
Siebertstraße 3
81675 München (DE)**

(54) **INSULIN RESISTANCE INHIBITOR FOR RUMINANT ANIMALS**

(57)    There is provided an agent for suppressing the insulin resistance of a ruminant, the agent including cashew nut shell liquid, heated cashew nut shell liquid, anacardic acid, cardanol, and/or cardol.

EP 4 052 708 A1

**Description**

BACKGROUND OF THE INVENTION

Field of the Invention

The present invention relates to an agent and feed for suppressing the insulin resistance of ruminants, and to a method of suppressing insulin resistance using the agent and the feed.

Description of the Related Art

In the case of keeping ruminants as domestic animals, the maintenance of the health conditions of the animals and measures against the diseases of the animals are important factors for improving the productivity of the animals.

Insulin resistance refers to a condition in which the action of insulin, such as an action of promoting sugar uptake, is deteriorated. The insulin resistance may cause diabetes mellitus, hyperlipidemia, or the like. In particular, perinatal ruminants have the risk of easily having insulin resistance due to a change in energy metabolism or hormone balance.

However, there have hardly been known feed components effective for suppressing insulin resistance in ruminants.

Known examples of the effects of cashew nut shell liquid on ruminants include the effect of improving rumen fermentation (Patent Document 1), the effect of preventing bloat (Patent Document 2), the effect of preventing coccidiosis (Patent Document 3), and the effect of preventing acidosis (Patent Document 4). However, there have ever been no findings on suppression of the insulin resistance of ruminants by cashew nut shell liquid.

[Prior Art Documents]

[Patent Documents]

Patent Document 1: WO 2008/149992
Patent Document 2: WO 2008/149994
Patent Document 3: WO 2009/139468
Patent Document 4: WO 2010/053085

SUMMARY OF THE INVENTION

Problems to be solved by the Invention

An object of the present invention is to provide a material for suppressing the insulin resistance of a ruminant.

Means to solve the problem

The inventors intensively studied to solve the above-described problems and consequently found that insulin resistance can be suppressed by administering cashew nut shell liquid (hereinafter, may be abbreviated as "CNSL") to a ruminant. The inventors completed the present invention on the basis of such findings.

That is, the present invention is as follows.

(1) An agent for suppressing insulin resistance of a ruminant, the agent comprising cashew nut shell liquid, heated cashew nut shell liquid, anacardic acid, cardanol, and/or cardol.
(2) The agent for suppressing insulin resistance according to (1), wherein the ruminant is cattle.
(3) The agent for suppressing insulin resistance according to (1) or (2), wherein the ruminant is a female ruminant.
(4) The agent for suppressing insulin resistance according to (3), wherein the female ruminant is a female ruminant between 30 days before calving and 30 days after the calving.
(5) The agent for suppressing insulin resistance according to any one of (1) to (4), the agent has an effect of reducing an insulin resistance index by 30% or more and thereby decreasing free fatty acid and/or ketone body levels in blood.
(6) A feed for suppressing insulin resistance of a ruminant, the feed comprising cashew nut shell liquid, heated cashew nut shell liquid, anacardic acid, cardanol, and/or cardol.

(7) The feed according to (6), wherein said feed has a crude protein content of 12% dry mass or more.

(8) The feed according to (6) or (7), wherein said feed has a neutral detergent fiber content of 20% dry mass or more.

(9) A method of suppressing insulin resistance, the method comprising administering cashew nut shell liquid, heated cashew nut shell liquid, anacardic acid, cardanol, and/or cardol to a nonhuman animal.

(10) A method of suppressing insulin resistance of a ruminant, the method comprising administering cashew nut shell liquid, heated cashew nut shell liquid, anacardic acid, cardanol, and/or cardol to a ruminant.

(11) The method according to (10), wherein the ruminant is cattle.

(12) The method according to (10) or (11), wherein the ruminant is a female ruminant.

(13) The method according to (12), wherein cashew nut shell liquid, heated cashew nut shell liquid, anacardic acid, cardanol, and/or cardol are administered at any time point between 30 days before calving and 30 days after the calving.

(14) The method according to (12), wherein cashew nut shell liquid, heated cashew nut shell liquid, anacardic acid, cardanol, and/or cardol are administered at any time point between 30 days before calving and time of the calving.

(15) The method according to any one of (10) to (14), wherein an administration period is at least 14 days.

(16) The method according to any one of (10) to (15), wherein cashew nut shell liquid, heated cashew nut shell liquid, anacardic acid, cardanol, and/or cardol are administered at 0.1 to 50 g/ruminant/day.

(17) Use of cashew nut shell liquid, heated cashew nut shell liquid, anacardic acid, cardanol, and/or cardol in manufacturing an agent for suppressing insulin resistance of a ruminant.

Effects of the Invention

In accordance with the present invention, insulin resistance can be suppressed by administering an agent or feed comprising cashew nut shell liquid, heated cashew nut shell liquid, anacardic acid, cardanol, and/or cardol to a ruminant. As a result, the health condition of the ruminant can be maintained to improve rearing efficiency.

DETAILED DESCRIPTION OF THE PREFERRED EMBODIMENTS

An agent for suppressing the insulin resistance of a ruminant of the present invention comprises cashew nut shell liquid (CNSL), heated cashew nut shell liquid, anacardic acid, cardanol, and/or cardol.

In the present invention, suppression of insulin resistance means suppression of a condition in which insulin action such as an action of promoting sugar uptake is deteriorated.

The insulin resistance can be evaluated based on a RQUIKI value described below. The lower value can be evaluated as the higher insulin resistance. Accordingly, if RQUIKI value is suppressed by administration of CNSL or the like as compared to non-administration, it can be considered that insulin resistance is suppressed. The administration of the agent for suppressing insulin resistance of the present invention preferably allows an index for insulin resistance, such as a RQUIKI value, to be reduced by 30% or more, more preferably by 50% or more, in comparison with the case of non-administration.

$$RQUIKI = 1/[Log\,(Glu) + Log\,(Insulin) + Log\,(NEFA)]$$

wherein Glu, Insulin, and NEFA represent glucose level (mg/dL), insulin level ($\mu$U/mL), and free fatty acid level (mmol/L) in blood, respectively.

Cashew nut shell liquid is an oily liquid contained in the nutshell of the cashew nut tree (*Anacardium occidentale* L.). Cashew nut shell liquid contains, as its components, anacardic acid, cardanol, and cardol. Anacardic acid is converted into cardanol by heat treatment. However, cashew nut shell liquid (heated cashew nut shell liquid) containing only cardanol and cardol due to heat treatment may also be used.

Cashew nut shell liquid (unheated) extracted by pressing cashew nut shells comprises 55 to 80% by mass of anacardic acid, 5 to 20% by mass of cardanol and 5 to 30% by mass of cardol, as described in J. Agric. Food Chem. 2001, 49, 2548-2551.

Heated cashew nut shell liquid obtained by heat treatment of unheated cashew nut shell liquid (for example, at 70°C or more, preferably 130°C or more), causing anacardic acid, which is a main component of unheated cashew nut shell liquid, to be decarboxylated and converted into cardanol, comprises 0 to 10% by mass of anacardic acid, 55 to 80% by mass of cardanol, and 5 to 30% by mass of cardol.

Cashew nut shell liquid can be obtained as a vegetable oil extracted by pressing cashew nut shells. Cashew nut shell liquid can also be obtained by dry distillation or solvent extraction of cashew nut shells. For example, cashew nut shell liquid can also be obtained by a method as described in Japanese Unexamined Patent Publication No.

H08-231410. A commercial product of cashew nut shell liquid may be used.

The agent for suppressing the insulin resistance of the present invention may comprise anacardic acid, cardanol, and/or cardol themselves, instead of cashew nut shell liquid.

Examples of anacardic acid include naturally-occurring anacardic acid, synthetic anacardic acid, and derivatives thereof. Moreover, a commercial product of anacardic acid may also be used. Anacardic acid can be obtained as described in Japanese Unexamined Patent Publication No. H08-231410 by extracting cashew nut oil from cashew nut shells treated with an organic solvent, subjecting the obtained cashew nut oil to, for example, silica gel column chromatography, and applying a mixed solvent of n-hexane, ethyl acetate and acetic acid with a varying ratio to the chromatography for the elution of the obtained cashew nut oil (see Japanese Unexamined Patent Publication No. H03-240721, Japanese Unexamined Patent Publication No. H03-240716, etc.).

Examples of cardanol include naturally-occurring cardanol, synthetic cardanol, and derivatives thereof. Moreover, cardanol to be used in the present invention can be obtained by decarboxylation of anacardic acid, a main component of cashew nut shell liquid.

Examples of cardol include naturally-occurring cardol, synthetic cardol, and derivatives thereof. Moreover, cardol to be used in the present invention can be obtained from purification of cashew nut shell liquid.

The contents of cashew nut shell liquid, heated cashew nut shell liquid, anacardic acid, cardanol, and/or cardol in the agent for suppressing insulin resistance of the present invention are preferably 0.1% by mass to 100% by mass, more preferably 0.5% by mass to 95% by mass, and particularly preferably 1% by mass to 90% by mass based on the total amount of the agent.

A stock solution of cashew nut shell liquid, heated cashew nut shell liquid, anacardic acid, cardanol, and/or cardol can be directly orally administered as the agent for suppressing insulin resistance of the present invention.

The agent for suppressing insulin resistance of the present invention may comprise a component such as an excipient which can be used in a feed or pharmaceutical product, such as, for example, lactose, sucrose, D-mannitol, $\alpha$-starch, starch, corn starch, crystalline cellulose, bentonite, silica gel, or light anhydrous silicic acid, as well as cashew nut shell liquid, heated cashew nut shell liquid, anacardic acid, cardanol, and/or cardol.

The agent for suppressing insulin resistance of the present invention may further comprise an optional component such as a component effective in promotion of the growth of a ruminant, a nutritional component, a component enhancing storage stability, or a coating component, as well as cashew nut shell liquid, heated cashew nut shell liquid, anacardic acid, cardanol, and/or cardol. Examples of such optional components include source materials of feeds, such as bran, alfalfa, and timothy, feed additives, food source materials, food additives, pharmaceutical product materials, and other supplement components used in supplements for animals (hereinafter referred to as "supplements"). Examples thereof include: living bacteria agents such as bacteria belonging to the genus *Enterococcus*, bacteria belonging to the genus *Bacillus*, and *Lactobacillus bifidus*; enzymes such as amylase and lipase; vitamins such as L-ascorbic acid, choline chloride, inositol, and folic acid; minerals such as potassium chloride, ferric citrate, magnesium oxide, and phosphates; amino acids such as DL- alanine, DL- methionine, and L-lysine; organic acids such as fumaric acid, butyric acid, lactic acid, and acetic acid, and salts thereof; antioxidants such as ethoxyquin, dibutyl hydroxytoluene, butylated hydroxyanisole, ferulic acid, vitamin C, and vitamin E; fungicides such as calcium propionate; binding agents such as carboxymethyl cellulose (CMC), sodium caseinate, and sodium polyacrylate; emulsifiers such as lecithin, glycerin fatty acid ester, and sorbitan fatty acid ester; coloring agents such as astaxanthin and canthaxanthin; and flavoring agents such as various esters, ethers, and ketones. The kinds of the supplements, and the components other than cashew nut shell liquid, heated cashew nut shell liquid, anacardic acid, cardanol, and/or cardol are not particularly limited.

The agent for suppressing insulin resistance of the present invention may comprise an oil absorption agent such as magnesium oxide, stearate, talc, zeolite, diatom earth, or silica, and the oil absorption agent is preferably granulous. The oil absorption agent is preferably an oil absorption agent that adsorbs 50 to 300 g of oil per 100 g of the oil absorption agent. When the oil absorption agent has a particle diameter of more than 300 $\mu$m, the particles of the oil absorption agent are coarsened and separated. Therefore, the oil absorption agent preferably has a particle diameter of 2 to 300 $\mu$m.

In one embodiment of the agent for suppressing insulin resistance of the present invention, a preferred mass ratio between the oil absorption agent and cashew nut shell liquid (CNSL), heated cashew nut shell liquid, anacardic acid, cardanol, and/or the cardol is 100:20 to 100:180. Moreover, a preferred mass ratio between the oil absorption agent and a cashew nut shell ground product is 15:100 to 60:100.

The dosage form for the agent for suppressing insulin resistance of the present invention is not particularly limited, but examples thereof include optional forms such as liquid medicine, powder, solid, tablet, capsule, emulsion, pellet, tablet, and coating. The dosage form is preferably liquid medicine, powder, capsule, pellet, or tablet.

Cashew nut shell liquid, heated cashew nut shell liquid, anacardic acid, cardanol, and/or cardol may be used on an as-is basis as the liquid agent, the liquid medicine in which cashew nut shell liquid, heated cashew nut shell liquid, anacardic acid, cardanol, and/or cardol are dissolved in a solvent such as ethanol may be used, or the liquid medicine to which the above-described excipient or an optional component is added may be used. The powder, capsule, pellet, or tablet described below may be suspended and allowed to float in a liquid.

As for the powder, the above-described excipient may be added to cashew nut shell liquid, heated cashew nut shell liquid, anacardic acid, cardanol, and/or cardol to make the powder.

As for the capsule, a capsule may be filled with cashew nut shell liquid, heated cashew nut shell liquid, anacardic acid, cardanol, and/or cardol, and optionally the above-described excipient or an optional component may be added to the capsule.

As for the pellet, the above-described excipient may be added to cashew nut shell liquid, heated cashew nut shell liquid, anacardic acid, cardanol, and/or cardol, and the resultant may be granulated to make the pellet.

As for the tablet, the above-described excipient may be added to cashew nut shell liquid, heated cashew nut shell liquid, anacardic acid, cardanol, and/or cardol, and the resultant may be granulated to make the tablet.

It is preferable to formulate the agent as the powder, the tablet, and the pellet when the agent comprises an oil absorption agent such as silica.

As described above, the agent for suppressing insulin resistance of the present invention can be produced by mixing cashew nut shell liquid, heated cashew nut shell liquid, anacardic acid, cardanol, and/or cardol, and an excipient or an optional component, as necessary, for formulation. In addition, according to the form of the agent, a product obtained by pulverizing/crushing the cashew nut shell, or cashew nut shell without being processed may be mixed directly with any other optional components to produce the agent for suppressing insulin resistance of the present invention. Furthermore, pulverized/crushed cashew nut shell itself, or cashew nut shell itself, not mixed with any other optional components, may be used as the agent for suppressing insulin resistance.

The feed of the present invention comprises cashew nut shell liquid, heated cashew nut shell liquid, anacardic acid, cardanol, and/or cardol.

The contents of cashew nut shell liquid, heated cashew nut shell liquid, anacardic acid, cardanol, and/or the cardol in the feed of the present invention are preferably 0.5 to 500,000 ppm by mass, more preferably 5 to 300,000 ppm by mass, and particularly preferably 50 to 100,000 ppm by mass based on the total amount per dry matter mass of the feed. When the contents are not less than 0.5 ppm by mass, the effect of suppressing insulin resistance can be expected. The tendency thereof is remarkable when the contents are not less than 5 ppm by mass, particularly preferably not less than 50 ppm by mass. In contrast, suppression of insulin resistance can be exhibited even when the contents are 500,000 ppm by mass. It is preferable that the contents are not more than 300,000 ppm by mass, particularly preferably not more than 100,000 ppm by mass.

In a preferred embodiment, the feed of the present invention has a crude protein (CP) content of 12% dry mass or more, 15% dry mass or more, or 17 dry mass or more. The upper limit of the crude protein content is not limited and, for example, the crude protein content is 30% dry mass or less. The CP content can be measured according to the Kjeldahl method (wet analysis).

In a preferred embodiment, the feed of the present invention has a neutral detergent fiber (NDF) content of 20% dry mass or more, 25% dry mass or more, or 30% dry mass or more. The upper limit of the neutral detergent fiber content is not limited and, for example, the neutral detergent fiber content is 50% dry mass or less. The NDF content can be measured according to the method of detergent analysis (wet analysis).

The analytical methods for CP and NDF are also shown in Feed Analysis Review (2004) published by Japan Scientific Feeds Association in March, 2004.

By increasing the content of CP and/or NDF in the feed of the present invention comprising cashew nut shell liquid, heated cashew nut shell liquid, anacardic acid, cardanol, and/or the cardol, the effect of suppressing insulin resistance can be synergistically enhanced while maintaining healthy conditions, especially for female ruminants just before and after calving.

The feed of the present invention can be produced by adding cashew nut shell liquid, heated cashew nut shell liquid, anacardic acid, cardanol, and/or cardol, or the agent for suppressing insulin resistance of the present invention comprising cashew nut shell liquid, heated cashew nut shell liquid, anacardic acid, cardanol, and/or cardol, to a feed component, and by mixing the resultant.

In such a case, when the agent which is powdery and solid is used, the agent may be allowed to be in liquid or gel form using a liquid carrier to facilitate the mixture. In this case, a flowable liquid such as water, vegetable oil, liquid animal oil, mineral oil, synthetic oil, or a water-soluble polymer compound can be used as the liquid carrier. Moreover, water-soluble polysaccharides such as alginic acid, sodium alginate, xanthan gum, carboxymethyl cellulose, $\alpha$-starch, sodium caseinate, gum arabic, guar gum, and tamarind seed polysaccharides are also preferably combined to keep the homogeneity of cashew nut shell liquid, heated cashew nut shell liquid, anacardic acid, cardanol, and/or the cardol in the feed.

For the feed of the present invention, the kinds, combination rates, and the like of feed components combined with the agent of the present invention are not particularly limited. The feed may be a feed conventionally given to animals. The feed can be prepared using, for example, corn grains, corn flours, milo, bran, soybean cake, oats, wheat short, wheat coarse powders, alfalfa, timothy, clovers, defatted rice bran, northern ocean meal, coast meal, yeast, molasses, meat pieces, bone meal, calcium carbonate, calcium diphosphate, yellow grease, vitamins, minerals, and/or the like.

The kinds of ruminants ingested with the agent for suppressing insulin resistance or feed of the present invention are not particularly limited, but examples thereof include ruminants such as cattle, buffalos, goats, sheep, and yaks. Among them, cattle are particularly preferably ingested. Preferred examples of the kinds of the cattle include female Holstein, Jersey, Japanese black, Japanese Shorthorn, and Aberdeen Angus. The amount of the agent for suppressing insulin resistance or feed to be given may be appropriately adjusted depending on the kind, body weight, age, sex, and health condition of the animal, feed components, and the like. In this case, the amounts of cashew nut shell liquid, heated cashew nut shell liquid, anacardic acid, cardanol, and/or cardol contained in the feed are, for example, 0.01 to 500 g/animal/day, preferably 0.1 to 200 g/animal/day, more preferably 0.1 to 50 g/animal/day, and still more preferably 0.5 to 5 g/animal/day.

Moreover, the range of the body weight of cattle ingested with the agent for suppressing insulin resistance or feed of the present invention is commonly 500 to 900 kg. Therefore, the amounts of cashew nut shell liquid, heated cashew nut shell liquid, anacardic acid, cardanol, and/or cardol contained in the feed given daily per kg of the body weight of cattle are 0.000011 g to 1 g, preferably 0.00011 g to 0.4 g, and more preferably 0.0011 g to 0.2 g.

The timing of administration of the agent for suppressing insulin resistance or feed of the present invention is not particularly limited; however, in the case of a milk cow, the agent or feed is preferably given to a milk cow having an age in month suitable for milk production (commonly an age of 20 to 144 months, preferably an age of 20 to 132 months, and more preferably an age of 20 to 108 months).

Moreover, the agent for suppressing insulin resistance or feed of the present invention is preferably given to a perinatal ruminant, preferably to a ruminant in a transitional period. For example, the agent or feed can be given at any time point between 60 days before calving and 30 days after the calving, preferably between 30 days before calving and 30 days after the calving, more preferably between 14 days before calving and 14 days after the calving. Particularly for suppressing insulin resistance resulting from calving, it is preferable to give the agent or feed from before calving, and, for example, the agent or feed can be given at any time point between 60 days before calving and the time of the calving, preferably between 30 days before calving and the time of the calving, more preferably between 14 days before calving and the time of the calving. In this case, the agent or feed may be given between before and after calving. A giving period is not particularly limited, but is preferably 14 days or more, more preferably 30 days or more, still more preferably 60 days or more.

EXAMPLES

The present invention will be specifically described below with reference to Example. However, aspects of the present invention are not limited to the following Examples.

Evaluation of Effect of Suppressing Insulin Resistance

Nine cows in total including five cows in the control group and four cows in the administration group were subjected to the study. A feed comprising 5.0 g of cashew nut shell liquid (containing 61.8% by mass of anacardic acid, 8.2% by mass of cardanol, and 19.9% by mass of cardol) was administered to the administration group from 14 days before calving. The CP content of the feed was over 12 dry mass % and the NDF content of the feed was over 20 dry mass %.

The cows tested were adult female Holstein cows (having an average age of 57.5 months, in a range between ages of 25 months to 90 months), which were individuals that were obese cows exhibiting a body condition score (BCS), indicating an obesity index, of 3.5 to 4.0, and that had the high risk of acquiring insulin resistance.

Blood was collected on 14 days before calving, and on 3 days and 7 days after the calving, to measure items related to insulin resistance and to inflammation in the body. The results are set forth in Table 1.

[Table 1]

| | | 14 days before calving | | 3 days after calving | | 7 days after calving | |
|---|---|---|---|---|---|---|---|
| | | Control | CNSL | Control | CNSL | Control | CNSL |
| Glucose (Glu) | mg/dL | 56.40±25.22 | 58.75±29.38 | 49.40±22.09 | 39.25±19.63 | 53.00±23.70 | 43.00±21.50 |
| Insulin (Insulin) | $\mu$ IU/mL | 2.64± 1.18 | 2.80± 1.40 | 0.52± 0.23 | 0.45± 0.23 | 0.92± 0.41 | 0.70± 0.35 |
| Non-esterified fatty acid (NEFA) | mEq/L | 0.14± 0.06 | 0.11 ± 0.06 | 0. 70± 0.31 | 0.55± 0.27 | 0.52± 0.23 | 0.56± 0.28 |
| beta-Hydroxybutyric acid (BHBA) | | 0.56± 0.25 | 0.55± 0.28 | 0.88± 0.39 | 0.90± 0.45 | 0.96± 0.43 | 1.00± 0.50 |
| ROUIKI [1] | | 0.81± 0.36 | 0.82± 0.41 | 0.84± 0.38 | 1.23± 0.62 | 0.75± 0.34 | 0.87± 0 44 |

†: There is a significant difference between the control and giving groups ($p < 0.05$).

<Results/Discussion>

On 3 days after the calving, there were differences between the measurement values, and the RQUIKI value was significantly high in the administration group, revealing that the insulin resistance was not exhibited in the administration group.

**Claims**

1. An agent for suppressing insulin resistance of a ruminant, the agent comprising cashew nut shell liquid, heated cashew nut shell liquid, anacardic acid, cardanol, and/or cardol.

2. The agent for suppressing insulin resistance according to claim 1, wherein the ruminant is cattle.

3. The agent for suppressing insulin resistance according to claim 1 or 2, wherein the ruminant is a female ruminant.

4. The agent for suppressing insulin resistance according to claim 3, wherein the female ruminant is a female ruminant between 30 days before calving and 30 days after the calving.

5. The agent for suppressing insulin resistance according to any one of claims 1 to 4, the agent comprising an effect of reducing an insulin resistance index by 30% or more and thereby decreasing free fatty acid and/or ketone body levels in blood.

6. A feed for suppressing insulin resistance of a ruminant, the feed comprising cashew nut shell liquid, heated cashew nut shell liquid, anacardic acid, cardanol, and/or cardol.

7. The feed according to claim 6, wherein said feed has a crude protein content of 12% dry mass or more.

8. The feed according to claim 6 or 7, wherein said feed has a neutral detergent fiber content of 20% dry mass or more.

9. A method of suppressing insulin resistance, the method comprising administering cashew nut shell liquid, heated cashew nut shell liquid, anacardic acid, cardanol, and/or cardol to a nonhuman animal.

10. A method of suppressing insulin resistance of a ruminant, the method comprising administering cashew nut shell liquid, heated cashew nut shell liquid, anacardic acid, cardanol, and/or cardol to a ruminant.

11. The method according to claim 10, wherein the ruminant is cattle.

12. The method according to claim 10 or 11, wherein the ruminant is a female ruminant.

13. The method according to claim 12, wherein cashew nut shell liquid, heated cashew nut shell liquid, anacardic acid, cardanol, and/or cardol are administered at any time point between 30 days before calving and 30 days after the calving.

14. The method according to claim 12, wherein cashew nut shell liquid, heated cashew nut shell liquid, anacardic acid, cardanol, and/or cardol are administered at any time point between 30 days before calving and time of the calving.

15. The method according to any one of claims 10 to 14, wherein an administration period is at least 14 days.

16. The method according to any one of claims 10 to 15, wherein cashew nut shell liquid, heated cashew nut shell liquid, anacardic acid, cardanol, and/or cardol are administered at 0.1 to 50 g/ruminant/day.

17. Use of cashew nut shell liquid, heated cashew nut shell liquid, anacardic acid, cardanol, and/or cardol in manufacturing an agent for suppressing insulin resistance of a ruminant.

## INTERNATIONAL SEARCH REPORT

International application No.

PCT/JP2020/040763

### A. CLASSIFICATION OF SUBJECT MATTER

A61K 31/60(2006.01)i; A23K 10/37(2016.01)i; A23K 20/158(2016.01)i; A23K
50/10(2016.01)i; A61K 31/05(2006.01)i; A61K 36/22(2006.01)i; A61P
3/10(2006.01)i
FI:     A61K31/60; A23K20/158; A61K36/22; A61K31/05; A61P3/10; A23K50/10;
        A23K10/37
According to International Patent Classification (IPC) or to both national classification and IPC

### B. FIELDS SEARCHED

Minimum documentation searched (classification system followed by classification symbols)
A61K31/60; A23K10/37; A23K20/158; A23K50/10; A61K31/05; A61K36/22;
A61P3/10

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2015/056729 A1 (IDEMITSU KOSAN CO., LTD.) 23 April 2015 (2015-04-23) claim 1, paragraphs [0019]-[0021] | 1-17 |
| Y | TEDONG, Leonard et al., "Hydro-ethanolic extract of cashew tree (Anacardium occidentale) nut and its principal compound, anacardic acid, stimulate glucose uptake in C2Cl2 muscle cells.", Molecular Nutrition & Food Research, 2010, vol. 54, no. 12, pp. 1753-1762 abstract, page 1757, column 3.4, fig. 4 | 1-17 |
| Y | US 5202355 A (TAKASAGO INSTITUTE FOR INTERDISCIPLINARY SCIENCE INC.) 13 April 1993 (1993-04-13) claim 1, column 5, example 1, table 1 | 1-17 |

☒ Further documents are listed in the continuation of Box C.          ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 17 December 2020 (17.12.2020) | 28 December 2020 (28.12.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2020/040763 |

**C (Continuation).   DOCUMENTS CONSIDERED TO BE RELEVANT**

| Category* | Citation of document, with indication, where appropriate, of the relevant passages | Relevant to claim No. |
| --- | --- | --- |
| P, X | CHUNG, Sang-Won et al., "Anacardic acid mitigates liver fat accumulation and impaired glucose tolerance in mice fed a high-fat and high-sucrose diet.", Food science & nutrition, February 2020, vol. 8, no. 2, pp. 796-804 entire text | 1-17 |
| A | OISHI, Katsutaka et al., "Wheat alkylresorcinols suppress high-fat, high-sucrose diet-induced obesity and glucose Intolerance by increasing insulin sensitivity and cholesterol excretion in male mice.", Journal of Nutrition, 2015, vol. 145, no. 2, pp. 199-206/1-199-206/8 entire text | 1-17 |
| A | KAMTCHOUING, Pierre et al., "Protective role of Anacardium occidentale extract against streptozotocin-induced diabetes in rats.", Journal of Ethnopharmacology, 1998, vol. 62, no. 2, pp. 95-99 entire text | 1-17 |
| A | JP 2014-139166 A (NATIONAL INSTITUTE OF ADVANCED INDUSTRIAL SCIENCE AND TECHNOLOGY) 31 July 2014 (2014-07-31) | 1-17 |
| A | CN 108056472 A (LUDONG UNIVERSITY) 22 May 2018 (2018-05-22) | 1-17 |

Form PCT/ISA/210 (continuation of second sheet) (January 2015)

INTERNATIONAL SEARCH REPORT

Information on patent family members

International application No.

PCT/JP2020/040763

| Patent Documents referred in the Report | Publication Date | Patent Family | Publication Date |
|---|---|---|---|
| WO 2015/056729 A1 | 23 Apr. 2015 | TW 201521592 A | |
| US 5202355 A | 13 Apr. 1993 | (Family: none) | |
| JP 2014-139166 A | 31 Jul. 2014 | (Family: none) | |
| CN 108056472 A | 22 May 2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008149992 A
- WO 2008149994 A
- WO 2009139468 A
- WO 2010053085 A

- JP H08231410 A
- JP H03240721 A
- JP H03240716 A

**Non-patent literature cited in the description**

- *J. Agric. Food Chem.,* 2001, vol. 49, 2548-2551